# EUROPEAN PATENT SPECIFICATION

(11) **EP 3 146 338 B1**
(45) Date of publication and mention of the grant of the patent: **19.06.2019**
(21) Application number: 15732450.0
(22) Date of filing: 07.04.2015
(51) Int. Cl.: G01N 33/543, G01N 33/569, G01N 33/68

(54) **METHOD FOR RAPID DETECTION OF INFECTION FROM CAGA POSITIVE HELICOBACTER PYLORI AND DIAGNOSTIC KIT THEREFOR**
VERFAHREN ZUM RASCHEN NACHWEIS EINER INFEKTION DURCH CAGA-POSITIVE HELICOBACTER PYLORI UND DIAGNOSTISCHER KIT DAFÜR
PROCÉDÉ DE DÉTECTION RAPIDE D'UNE INFECTION PAR UNE SOUCHE CAGA POSITIVE D'HELICOBACTER PYLORI, ET KIT DE DIAGNOSTIC ASSOCIÉ

(30) Priority: 04.04.2014 IT RM20140179
(43) Date of publication of application: 29.03.2017
(73) Proprietor: Over S.r.l., 53100 Siena (SI) (IT)
(72) Inventor: FIGURA Natale, I-53100 Siena (IT)
(74) Representative: Banchetti, Marina
(86) International application number: PCT/IT2015/000101
(87) International publication number: WO 2015/151128

(56) References cited:
- EP-A1- 1 227 159
- CN-A- 102 393 460
- BLANCO S ET AL: "Evaluation of a latex agglutination test (PYLOGEN) for the detection of Helicobacter pylori in stool specimens", DIAGNOSTIC MICROBIOLOGY AND INFECTIOUS DISEASES, ELSEVIER SCIENCE PUBLISHING CO., AMSTERDAM, NL, vol. 63, no. 4, 1 April 2009 (2009-04-01), pages 349-353, XP026007280, ISSN: 0732-8893, DOI: 10.1016/J.DIAGMICROBIO.2008.12.006 [retrieved on 2009-02-18]
- K H Wong ET AL: "Typing of heat-stable and heat-labile antigens of Campylobacter jejuni and Campylobacter coli by coagglutination", Journal of Clinical Microbiology, 1 May 1985 (1985-05-01), pages 702-707, XP055157282, UNITED STATES Retrieved from the Internet: URL:http://jcm.asm.org/content/21/5/702.ab stract [retrieved on 2014-12-09]
- AIKO YASUDA ET AL: "A novel diagnostic monoclonal antibody specific for Helicobacter pylori CagA of East Asian type", APMIS, vol. 117, no. 12, 17 November 2009 (2009-11-17), pages 893-899, XP055157605, ISSN: 0903-4641, DOI: 10.1111/j.1600-0463.2009.02548.x
- "Rapid test in card format for detecting Helicobacter pylori antigen in stool specimen", , 28 March 2012 (2012-03-28), XP055157612, Retrieved from the Internet: URL:http://www.clonit.it/en-GB/products/po int-of-care/ [retrieved on 2014-12-09]

## Description

### Field of the invention

The present invention concerns a method for the rapid detection of infection from CagA-positive *Helicobacter pylori,* and a diagnostic kit for carrying out said method. More specifically, the invention concerns an *in vitro* method for detecting, in samples of gastric mucus, stools or biopsy specimens, the presence of highly pathogenic CagA strains of *H. pylori,* which method provides results immediately available on performing the test, as well as the diagnostic kits for use in carrying out said method.

### Background of the invention

As is known, *Helicobacter pylori* is a helix-shaped Gram-negative microaerophilic microorganism that can inhabit the stomach and the duodenum, adapting itself to the inhospitable environment of the gastric mucosa in a quite particular way, to the point that it often fails to produce any detectable symptoms. Actually, it is considered that more than 80% of the individuals infected with *H. pylori* are asymptomatic. Such microorganism infects more than half of the world population at the level of the gastro-duodenal tract. The transmission route of the infection has not been clarified, although infection seems to be typically acquired in the childhood.

The stomach is protected from its own gastric juice, made of concentrated hydrochloric acid and rich of digestive enzymes, by a thick layer of mucus which covers the gastric mucosa, within which the *Helicobacter* may penetrate and survive, resisting to any acid that may possibly reach it thanks to an enzyme produced by such bacterium, urease. Urease converts the urea contained in large amounts in the stomach into bicarbonate and ammonium, therefore a basic environment is established in the vicinity of the bacterial colony, such environment being capable of neutralizing the hydrochloric acid of gastric secretions, thereby protecting *H. pylori.*

Another protective mechanism available to *Helicobacter pylori* consists in that the natural immune defences of the body cannot reach the microorganism in the gastric mucus. The immune system reacts to *H. pylori* infection by sending leukocytes, T killer lymphocytes and other defence factors, but these cannot reach the infection site, as they cannot easily penetrate through the mucus layer, and remain and accumulate in the site, leaving there their destructive content (consisting of superoxide radicals) when they die. Additional nutrients are sent on the site to assist the white blood cells, and this fact does no more than further feeding *Helicobacter* itself. As a result, gastritis develops in a few days, and then it may sometimes progress up to peptic ulcer. In view of the foregoing, it is considered that the causative agent of the damage to the gastric mucosa is not *H. pylori* itself, but the inflammation and, consequently, the immune response to the microorganism.

From the scientific literature it appears that infection from *Helicobacter pylori* is the main cause of chronic gastritis and the most relevant factor for the development of peptic ulcer (Warren JR, Marshall BJ. Unidentified curved bacilli on gastric epithelium in active chronic gastritis. Lancet 1983; 1:1273-5). By colonizing the stomach, *H. pylori* induces a persistent inflammation (chronic gastritis) which may last years without developing with clinical symptoms. However, in 10-20% of the cases, chronic gastritis may result in the development of gastric or duodenal ulcers.

Also gastric cancer (gastric adenocarcinoma and non-Hodgkin gastric lymphoma) is often associated with *H. pylori* (Moss SF. The carcinogenic effect of H. pylori on the gastric epithelial cell. J Physiol Pharmacol 1999; 50:847-56). In the frame of a wide review of cases of gastric carcinoma it has been shown that the presence of *H. pylori* results in a six-fold increase of the risk of gastric cancer. It is believed that chronic gastritis may lead to intestinal metaplasia, which in turn, may degenerate into malignant cancer. The low grade malignant lymphoma o MALToma, in turn, seems to derive from the malignant degeneration of lymphoid tissue associated with the mucosa. In this case, retrospective bioptic studies demonstrated that 90% of these MALTomes are associated to the presence of *H. pylori.*

In addition to the mentioned gastroduodenal diseases, infection with H. *pylori* can aggravate or contribute to the development of coronary heart disease (ischemic heart disease up to myocardial infarction), metabolic disorders (such as increased LDL cholesterol and HDL cholesterol reduction, insulin resistance, etc.), autoimmune diseases (such as autoimmune thyroiditis, systemic sclerosis, etc.) and several other conditions such as osteoporosis, reduced male and female fertility, etc.. For all these reasons, it is necessary to detect early infection and treat it appropriately.

The diagnosis of *H. pylori* infection is commonly done through the research of bacterial urease in gastric biopsies, during endoscopy, or by looking for bacterial antigens in stool samples. Gastrointestinal endoscopic examination is certainly more reliable because it allows direct viewing of esophageal and gastric mucosa, removal of material by biopsy and culture of the material removed for *H. pylori.* However, it is an invasive and rather expensive test.

The search of anti-Hp antibodies in blood would represent a cheap and fairly rapid method of diagnosis, advantageous due to the fact that the detection of specific IgGs in serum has good sensitivity and good specificity. However, its utility is severely limited, as it does not distinguish between active infection and past infection. In fact, the anti-helicobacter antibodies can circulate in the bloodstream for more than two years after recovery from the infection.

The search for fecal antigen of *H. pylori,* HpSA, may identify the presence of antigen in stool and, since this is a sign of an active infection, this test is more reliable than detection of antibodies in the blood. However, with this type of survey the level of false negatives is remarkable, therefore the test is safe only when it is positive. A known method of rapid diagnosis based on detection of the fecal *H. pylori* antigen employs an analytical technique known as coagglutination test, in its variant known as "latex agglutination", which uses polymeric latex particles.

The original method of coagglutination (Kronvall, G. 1973. A rapid slide agglutination method for typing pneumococci by means of specific antibody absorbed to protein A-containing staphylococci. J. Med. Microbiol. 6: 187-190) is based on a Staphylococcus strain, the strain of *Staphylococcus aureus* Cowan 1, which is rich in protein A, a substance having the characteristic of adsorbing in a specific, but non-immunological, manner, immunoglobulins G (IgGs) of subclasses 2 and 4, linking the Fc fragment and leaving the Fab portion free to interact with a variety of antigenic molecules. Staphylococci coated with antibodies thus become a reagent which agglutinates in the presence of the homologous antigen. Agglutination of this *S. aureus-protein* A complex can be used to detect the presence of antigens (bacterial, viral and parasitic antigens), in body fluids, and for the immunological identification of various pathogens.

The scientific article by K. H. Wong et al., "Typing of heat-stable and heat-labile antigens of Campylobacter jejuni and Campylobacter coli by coagglutination"(J. Clin. Microbiol., 21(5), 1985, pp.702-707) discloses the performance of the known coagglutination method for the detection of *H. pylori* (*Campilobacter*) infection using a *Staphylococcus aureus* Cowan strain.

In the variant known as latex agglutination test staphylococci are replaced by polymer microspheres of uniform size (typically with a diameter of 8 µm) obtained by emulsion polymerization of hydrocarbon monomers. The microspheres, known in the field with the name of "latex" particles, exhibit, on their surface, the hydrophilic heads of the hydrocarbon chains that constitute them. These are coated with antisera of bacterial, viral or other origin in a manner similar to that described for staphylococci suspensions, and then the latex sensitization process is similar to that previously described for staphylococci containing Protein A. Therefore, the rapid diagnostic method mentioned above (Blanco et al.,"Evaluation of a latex agglutination test (PYLOGEN) for the detection of Helicobacter pylori in stool specimens", Diagn Microbiol Infect Dis. 2009; 63: 349-353) is based on putting in contact a suspension of latex particles sensitized with a monoclonal antibody directed specifically against *H*. *pylori* antigens with a stool sample of the subject to be analyzed, and visually checking the possible agglutination, which would be indicative of the presence of *H. pylori* in the sample.

It should be noted that the *H. pylori* antigens used for sensitization of the latex particles in the test mentioned above are of polysaccharide nature. According to current knowledge, it is presumed that the different *H. pylori* strains will not be able to differentiate between fully virulent (because associated with ulcers and cancer) and less virulent on the basis of the constitution of their polysaccharide. In addition, as is evident from the work mentioned above, the main indication of Pylogen is the verification of the eradication treatment, because its sensitivity (75%) in the post-treated patient is slightly better than that obtained in the pre-treatment diagnosis (73%), although the specificity compared to UBT (Urea Breath Test, described below) is less good (93% vs. 100%).

The examination for H. pylori infection currently regarded as the "gold standard" is the breath test with ¹³C-labeled urea (Urea Breath Test), which is still performed in a few centers, mainly because of its cost. The examination must be performed after an overnight fast, and involves the oral assumption of sodium citrate, followed, after ten minutes, by an examination of the air exhaled by the patient. The patient is then administered one tablet of radiolabelled urea, and a new sample of exhaled air is collected after about 30 minutes. Since, as noted, *H. pylori* splits urea into ammonia and bicarbonate, the amount of marked CO₂ exhaled after half an hour from a subject with ongoing infection will be higher than that exhaled from a healthy subject.

It is also known that the various strains of *Helicobacter pylori* do not all have the same pathogenic power: the strains having in their chromosome an insertion defined "cag pathogenicity island" (cag PAI), a set of genes involved in the virulence of the microorganism, exhibit a higher inflammatory potential. Approximately 50-70% of the strains of *H. pylori* found in Western countries contain the cag pathogenicity island, and patients infected with bacteria whose genome contains the insertion have a stronger gastric inflammatory response and a greater risk of developing peptic ulcers or gastric cancer than patients infected with strains that do not contain this insertion into their genome. The cag-positive strains are able to inject an oncoprotein (encoded by one of the cag genes) into colonized gastric cells, named CagA, which increases the risk of developing neoplastic lesions (Censini S, Lange C, Xiang Z, Crabtree JE, Ghiara P, Borodovsky M, et al. cag, a pathogenicity island of Helicobacter pylori, encodes type I-specific and disease-associated virulence factors. Proc Natl Acad Sci USA 1996; 93:14648-53).

Actually, it is to be considered that *H. pylori* is a microorganism that has evolved with man. It is part of the genus Helicobacter with thirty different species that have found their habitat in the stomach of many mammals and in intestines of many vertebrates, mammals and not (e.g. birds). The presence of *H. pylori* had to confer a selective advantage to individuals hosting it, otherwise this organism would have been lost in the evolutionary path. It is believed that hundreds of thousands of years ago, *H. pylori* was infected by a phage which inserted in its chromosome a DNA fragment taken from a bacterial species that no longer exists (because the nucleotide sequence of this segment is not found in any of the microorganisms examined so far). The insertion contains 30 genes involved in virulence, and is called "pathogenicity island" (PAI).

Other PAIs were already known in various bacterial species infecting humans and plants, such as *Yersinia enterocolitica* subsp. *pestis, Bordetella pertussis, Agrobacterium tumefaciens*: the genes which make up the various PAIs show a very high homology with each other, and all encode for the production of a species of conjugation factor, through which CagA, a protein considered to be an immune-dominant determinant, it is translated, almost injected, into the eukaryotic cell colonized. Once inside the cell, the CagA protein is phosphorylated in its tyrosine residues and transformed into an oncoprotein, able to alter the cell morphology and thus disturb the "signaling" systems of the host in such a way that can lead, ultimately, to the development of a gastric carcinoma. The carcinogenicity of CagA is demonstrated by the observation that transgenic mice expressing CagA systemically develop gastric carcinoma or tumors of the hematopoietic system.

In addition to the CagA protein, the bacterium also transfers part of its peptidoglycan, a constituent of its wall which has the ability to stimulate a strong inflammatory response mediated by transduction of the signal to which NF-πB participates. Inflammatory damage is added to the damage caused by CagA (which also has a strong pro-inflammatory power), and this combined action explains why patients with stomach carcinoma are, or were. all, with very few exceptions, infected with strains of *H. pylori* expressing CagA.

Studies on the polymorphism of the CagA protein have also shown that strains with mutilating deletions of the PAI or with few genomic repetitions of the variable region of *cagA* (encoding the amino acid sequence Epiya - Glu-Pro-Ile-Tyr-Ala - which is the one that is phosphorylated) are isolated less frequently in cases of gastric carcinoma, but rather increase the risk of peptic ulcer disease. The discovery of CagA-negative strains of *H. pylori* is exceptional in patients with ulcer or cancer. In other words, strains which host the PAI, of which CagA is the marker, can determine both peptic ulcer and gastric carcinoma. What makes the difference, apart from the host's inflammatory haplotype, is the polymorphism of *cagA* and/or the PAI genotype.

The foregoing observations have almost immediate practical effects, as they suggest that the diagnosis of *H. pylori* infection, as currently performed, is insufficient and incomplete in the absence of a characterization of the infecting strain CagA status. In fact, the risk of such diseases is high if the infecting bacteria host CagA, and is low if they are cagA-free.

As to the effects of CagA on extra-gastric diseases, the following observations have recently been made (Figura N, Franceschi F, Santucci A, Bernardini G, Gasbarrini G, Gasbarrini A. Extragastric manifestations of Helicobacter pylori infection. Helicobacter. 2010 Sep.15 Suppl 1:60-8):
1. Ischemic heart disease. Infection with CagA-positive strains is significantly more frequent in patients than in controls without cardiovascular disorders. The patients infected with CagA-positive strains also have higher cardiovascular damage, as evidenced by stress ECG, and serum levels of BNP (B-type Natriuretic Peptide) significantly higher (Figura N, Palazzuoli A, Vaira D, Campagna M, Moretti E, lacoponi F, Giordano N, Clemente S, Nuti R, Ponzetto A. Cross-sectional study: CagA-positive Helicobacter pylori infection, acute coronary artery disease and systemic levels of B-type natriuretic peptide. J Clin Pathol. 2014 Mar; 67(3):251-7).
2. Autoimmune thyroid disease. Infection with CagA-positive *H. pylori* is more prevalent in patients than in controls; moreover, it was also shown that eradication of the infection determines the disappearance of anti-thyroglobulin and anti-peroxidase antibodies.
3. The transaminases are higher (but still within the normal range) in patients who host a strain expressing CagA.
4. Patients with duodenal ulcers have higher concentrations of monocytes and polymorphonucleates if they have anti-CagA antibodies.
5. The frequency of infection by CagA-positive strains is higher in patients with osteoporosis than in controls, and anti-CagA antibody titers correlate in an almost linear fashion with the risk of pathological fractures in these patients.
6. Patients and individuals with anti-CagA antibodies are significantly more frequently infertile, both male and females, and males have a reduced percentage of sperm with reduced motility, and a high level of systemic inflammatory cytokines (Moretti E, Collodel G, Mazzi L, Campagna MS, Figura N. CagA-positive Helicobacter pylori infection and reduced sperm motility, vitality, and normal morphology. Dis Markers. 2013; 35(4):229-34).

Considering the diagnostic methods currently in use to detect the infection by *Helicobacter pylori,* the serologic method is, at the present time, the only method able to evidence an infection by the most virulent strains, since it allows to detect the presence of anti-CagA antibodies in the blood. However, as these antibodies can continue to circulate in the blood for as long as 10 years before going back to levels below the detection threshold of the common commercial tests, it is evident that a diagnosis of *H. pylori* infection allowing to detect high risk strains when the infection is in place is not possible through this method.

The Chinese patent application CN 102 393 460 A (Wang Jicheng) discloses monoclonal anti-CagA antibodies for the detection of *H. pylori* infection in stool specimens using a test paper, thereby suggesting that the CagA protein is closely associated with the pathogenicity of *H. pylori* and the severity of the related diseases.

In the light of the foregoing, it emerges that there does not exist, to date, a method suitable to detect the presence of *H. pylori* strains expressing CagA, or to detect the only CagA protein antigen, in biological samples taken from patients in an extemporaneous test, or in a test being quickly performable and having good reliability. The availability of such a diagnostic method would allow to carry out a rapid screening in order to identify which subjects, among those infected with *H. pylori* in place, are affected by CagA-positive strains, and thus are at higher risk of critical development of the pathology.

### Summary of the invention

According to the present disclosure, it is possible to rapidly discriminate, and with great sensitivity, between infections from CagA negative *H. pylori* and infections from CagA-positive *H. pylori* by applying the known analytical technique known as coagglutination test, both in its variant employing the *Staphylococcus aureus* Cowan 1 strain, and in its variant employing polymeric latex particles.

In the frame of the studies that led to the present invention, the possibility of sensitizing a latex polymer with an anti-CagA serum obtained by immunizing rabbits with a recombinant fragment from the CagA protein, whose gene had been cloned in *Escherichia coli,* was first considered. This method, although specific, did not prove to be very sensitive, probably due to the low concentration of antibodies in the serum.

In fact, the number and concentration of antigens required to produce a hyperimmune serum are both important factors, as it is also demonstrated by the observation that the latex sensitization with purified IgGs obtained from rabbits immunized with total protein antigens of *H. pylori* gives rise to a quick and highly visible agglutination when the amount of IgGs used is adequate, but if the amount of IgGs is just halved the quantity of IgGs sensitizing agglutination is considerably less evident.

In order to overcome the problem of a not optimal sensitivity of the above-described method which uses latex particles sensitized with anti-CagA antiserum, purified IgGs anti-total protein of *H. pylori* were produced, which were subsequently adsorbed with a suspension of a non-cytotoxic CagA-negative *H. pylori* strain. In this way, the serum was deprived of antibodies directed against antigens different from CagA and from other peptides encoded by the genes of the pathogenicity island (about 30) located upstream from *cagA.* It has been considered, in fact, that CagA is only one of the proteins encoded by the genes of the pathogenicity island *cag* (which contains about 30 "open reading frames"), each of which encodes for a peptide provided, as CagA, with a high immunogenicity. Furthermore, studies performed in the 90's have shown that, in a relevant portion of the isolated strains, *cag* is composed by two sections, right and left with respect to an insertion known as *IS605,* which divides it into two parts without interrupting it. Its presence causes instability of *cag,* resulting into a possible deletion of an arm of the island. These deletions reduce the virulence of the strains; it happens for some point mutations of genes located in the left or right branch, such as *cagE* and *virB10.*

As an evidence of the foregoing, a study conducted by the research group of the author of the present invention showed that the long-term prognosis (15 years) of cases of gastric cancer is much better when the infecting strain is free from the *cagE* and/or *virB10* genes.

Using in the coagglutination test an antiserum against total proteins of CagA+ *H. pylori* deprived of the other antibodies against *H. pylori* antigens different from those which characterize the pathogenicity island, in particular obtained by adsorbing the anti-total protein of CagA+ *H. pylori* antiserum with a suspension of a CagA-negative strain of *H. pylori,* it was possible to obtain, according to the invention, a good agglutination, equally specific not only for the CagA protein, but also for all other high virulence proteins of the cag pathogenicity island of *H. pylori* Type I (CagA positive and cytotoxic).

According to the present invention there is provided, therefore, a rapid diagnostic method for the detection of *H. pylori* strains expressing the *cag* pathogenicity island genes, particularly CagA-positive *H. pylori* strains, which method exploits the method of latex agglutination, in which latex particles sensitized with an antiserum anti-total proteins of CagA-positive *H. pylori* from which other antibodies against *H. pylori* antigens have been separated, in suitable conditions, with a biological sample to be analyzed, and the agglutination or not of the resulting suspension is detected, according to whether or not CagA antigens, and other high virulence proteins of the *cag* pathogenic island, are present in the sample.

The proposed method according to the invention can be applied, with minor modifications, to any type of sample in which it is necessary to quickly detect the presence of the CagA antigen, or of more proteins encoded by the *cag* genes, such as blood, stool, gastric biopsies, gastric mucus or other fluids (e.g., water, in order to determine the environmental contamination, etc.).

### Detailed description of the invention

Therefore, the present invention specifically provides an *in vitro* method for the rapid detection of infection from highly pathogenic *Helicobacter pylori* comprising the following steps:
a) contacting a suspension of latex particles, sensitized with an antiserum anti-total protein of CagA-positive *H. pylori* from which the other antibodies against *H. pylori* antigens have been separated, with a biological sample to be analyzed for the presence of the CagA protein antigen or of protein antigens encoded by the *cag* genes of *H. pylori,* and mixing said suspension with said biological sample;
b) visually detecting the possible agglutination of the mixture resulting from the previous step;
wherein the agglutination of said mixture shows the presence, in the biological sample under examination, of highly pathogenic *Helicobacter pylori* strains, which express the CagA protein or more proteins encoded by the cag genes.

According to some specific embodiments of the invention, said biological sample may consist of gastric mucus, stools or of a biopsy specimen of the gastric mucosa.

According to some preferred embodiments of the invention, the above step a) consists of contacting a suspension of latex particles sensitized with an antiserum anti-total protein of CagA-positive *H. pylori* subsequently adsorbed with a strain of CagA-negative *H. pylori* with a biological sample to be analyzed for the presence of the CagA protein antigen or of protein antigens encoded by *cag* genes, and mixing said suspension with said biological sample.

Preferably, according to the invention, the visual detection b) is carried out with the aid of a positive control sample consisting of a suspension of CagA-positive *H. pylori* which is subjected, as the biological sample to be analyzed, to operations corresponding to steps a) and b).

According to other preferred embodiments of the invention, the visual detection b) is carried out with the aid of a negative control sample, consisting of a suspension of latex particles sensitized or not sensitized with hyperimmune serum, which is used in place of the suspension of the step a) when subjecting the biological sample to be analyzed to steps corresponding to the steps a) and b).

In the specific case in which it is also requested to simultaneously detect the presence in the sample of any generic *H. pylori* strains, the method according to the invention may further comprise the following steps:
c) contacting a suspension of latex particles, sensitized with antiserum against total *H. pylori* antigens, with a biological sample to be analyzed for the presence of *H. pylori* antigens, and mixing said suspension with said biological sample; and
d) visually detecting the possible agglutination of the mixture resulting from the previous operation;
wherein the agglutination of said mixture shows the presence, in the biological sample under examination, of *Helicobacter pylori* strains, and
wherein the combined performance of the mentioned steps a), b, c) and d) allows to detect both a possible infection by H. *pylori* and the possible presence of highly pathogenic *Helicobacter pylori* strains.

To sensitize latex particles of varying diameter, 0.1 mL of undiluted antiserum is added to 1 mL suspension of latex particles, and allowed to react for a sufficient time, for example, at least 30 minutes. Then, the suspension of sensitized latex particles is diluted up to 10 mL with PBS and stored at 4°C until use.

The anti-CagA serum to sensitize latex particles may be obtained by adsorbing from the serum of a rabbit or other animal immunized with a strain of CagA-positive *H. pylori* the antibodies directed against helicobacter antigens, except the anti-CagA antibodies. This can be done by using a suspension of CagA-negative helicobacter: after adsorption, only anti-CagA antibodies remained in the serum.

It should be noted that the tests according to the invention do not require a reading carried out in laboratory: they are of immediate detection in the gastroenterologist's office. In practice, a quite evident agglutination is observed in less than one minute; in order to define a negative test, it necessary to wait for a couple of minutes. The negative control is represented by a suspension of staphylococci or latex particles not sensitized, or sensitized with non-specific immunoglobulins, which, obviously, must not agglutinate.

The test proposed according to the invention can be carried out, in particular, both on samples of gastric mucus and in stool samples.

### Version on gastric mucus:

According to a specific embodiment, the test is performed in the following way: a biopsy sample is dissolved in a drop of dithiothreitol, preferably 0.1% in PBS (dithiothreitol has the ability to open the disulfide bridges of the mucoproteins, thus thinning the mucus); one drop of a 1% suspension of latex particles sensitized with anti-CagA antiserum is added and the mixture is stirred with a stick.

If the sample contains CagA-positive helicobacter, a very apparent agglutination is observed in less than one minute. The test result is evaluated after waiting, preferably, for two minutes. For greater certainty, the test is preferably performed with a negative control, which consists of a suspension of non-sensitized latex particles, or of an iperimmune serum (with non-specific immunoglobulins) which, obviously, must not agglutinate when mixed with the test sample.

According to some preferred embodiments of the proposed method, a suspension of latex particles sensitized with total antigens of *H. pylori* can also be added in the test, and suspensions of latex particles sensitized with an antiserum against total antigens of helicobacter can be colored with a predetermined color, while suspensions of latex particles sensitized with anti-CagA antiserum can be colored with a different color. In this way, both the infection and the genotype of helicobacter are diagnosed with a single test. Latex particles sensitized with antiserum against total antigens of helicobacter may be colored, for example, in red, while those sensitized with anti-CagA antiserum may be colored in blue.

The test according to the invention can be also carried out on samples of gastric mucus collected through a silk or cotton thread glued to a capsule that is swallowed; the capsule, as it proceeds along the esophagus and stomach, drags the thread behind it. The upper end of the thread is positioned on the cheek with a patch. Once arrived in the stomach, the capsule dissolves on contact with hydrochloric acid; after 10-15 minutes, the thread, strongly impregnated with mucus at the distal end, is retrieved; the mucus is recovered and the test is carried out

According to a further aspect thereof, the present invention concerns a diagnostic kit for the *in vitro* rapid diagnosis of infection by highly pathogenic strains of *Helicobacter pylori,* comprising the following components:
A. a suspension of latex particles, sensitized with an antiserum against CagA-positive *H. pylori* from which the other antibodies against *H. pylori* antigens have been separated, in an aqueous solution such as phosphate-buffered saline (PBS) or PBS-formalin, containing an antimicrobial agent, such as merthiolate or sodium azide;
B. an aqueous solution such as PBS or PBS-formalin, containing a mucus-fluidifying agent, such as dithiothreitol, and an antimicrobial agent, such as m sodium merthiolate or sodium azide;
C. some cardboard sheets with dark disks, on which the test is to be performed.

According to a preferred embodiment of the invention, the proposed diagnostic kit also includes:
D1. a suspension of CagA-positive *H. pylori* antigens, in an aqueous solution such as PBS or PBS-formalin, and an antimicrobial agent, such as merthiolate or sodium azide, as positive control for CagA-positive *H. pylori.*

According to a further preferred embodiment of the invention, the proposed diagnostic kit also includes:
D2. a suspension of *H. pylori* antigens, in an aqueous solution such as PBS or PBS-formalin, and an antimicrobial agent, such as merthiolate or sodium azide as a positive control for *H. pylori.*

Preferably, in the proposed diagnostic kit according to the invention, the component B contains dithiothreitol in a concentration comprised between 0.05 and 1% by weight; in addition, said aqueous solution is a phosphate buffered saline (PBS) or a phosphate buffered saline containing 0.5-1.0% by weight of formalin (PBS-formalin), and said antimicrobial agent is merthiolate in a concentration of about 0.05% by weight or sodium azide in a concentration of about 0.1% by weight

### Composition of the kit

A specific embodiment of a kit for the rapid diagnosis of *H. pylori* infection consists of the following elements:
- a suspension in PBS of an antiserum against CagA-positive *H. pylori* from which the other antibodies against antigens of *H. pylori* have been separated;
- a PBS solution at pH 7.4 with dithiothreitol (or other mucus-thinning agent) (to avoid contamination and kill *H. pylori*);
- a suspension of CagA-positive *H. pylori* treated with formalin as positive control for highly pathogenic strains (with anti-CagA antibodies).

All mentioned components also contain a suitable concentration of merthiolate (thimerosal or, sodium ethylmercury-tiosalicilato), preferably to 0.05% by weight, or another equivalent antimicrobial agent.

According to a preferred embodiment of the diagnostic kit proposed according to the invention, the package contains the following:
- a flask of a first color, for example blue, with a suspension of anti-CagA abtibodies in PBS, with merthiolate as preservative (test suspension);
- a flask of a second color, for example white, with a solution of 0.1% dithiothreitol in PBS with merthiolate as preservative (mucus thinner suspension);
- a flask of a third color, for example red, containing total *H. pylori* antigens (for example, sonicated) in PBS and merthiolate, or in PBS-formalin (positive control for *H. Pylori*);
- some cardboard sheets with dark disks, on which the test is to be per-formed.

Dithiothreitol can be used in concentrations from 0.05 to 1% by weight, and is preferably 0.1% by weight.

As an alternative to dithiothreitol, other known dissolvers of mucus may be used, such as those used as mucolytic agents in the pharmaceutical industry, including, in particular, the acetylcysteine.

As antimicrobial agents merthiolate can be used, for example, sodium azide, preferably 0.1%.

As a positive control, in an alternative to suspension of *H. pylori* for-treated with formalin, it may be foreseen, according to the invention, the use of antigens of *H. pylori* extracts by means of ultrasonication or with glycine or with glucopiranoside (drastic detergent), in PBS containing sodium azide or merthiolate.

### Version on stool samples:

In the case of tests to be performed on stool samples, the diagnostic kit according to the invention should also contain a vial containing PBS with an antimicrobial agent, preferably merthiolate, and a protease inhibitor (to prevent proteases from digesting protein antigens of *H. pylori*). After suspending a small amount of stool in this solution, the suspension is left to settle; then a small amount of supernatant is taken, which is used for the assay.

The specific features of the invention, as well as the advantageous aspects thereof, will become more apparent with reference to the detailed description presented merely by way of example in the following, together with the results of the experimentation carried out on it.

### Brief description of the drawings

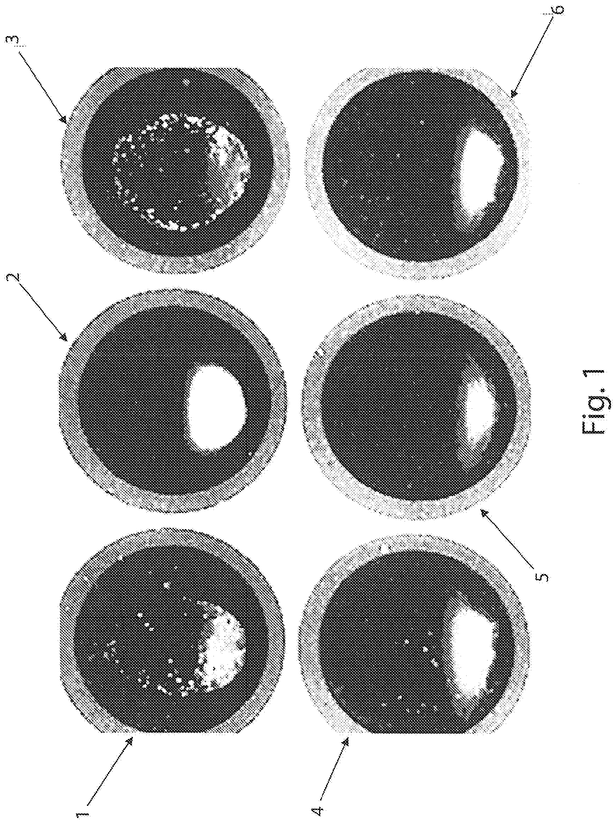

**Figure 1** shows photographic images of the cardboards of a first version of the diagnostic kit according to the invention, with the results of the latex agglutination test for detection of strains of CagA-positive *H. pylori,* in which the following results are visible:
   wells 1 and 3: positive latex agglutination; the well 1 contains gastric mucus dissolved with dithiothreitol and mixed with a drop of latex sensitized with anti-CagA+ *H. pylori*; the well 3 contains a mixture of gastric mucus dissolved with dithiothreitol and mixed with a drop of latex sensitized with antibodies against total antigens of CagA+ *H. pylori* (positive control) - in the case shown the test is positive for CagA+H. *pylori*;
   well 2 contains a mixture of gastric mucus taken from a non-infected patient, in dithiothreitol, and sensitized latex; the test is negative;
   wells from 4 to 6 contain sensitized latex and an amount of agar culture of *Helicobacter felis, Helicobacter mustelae* and *Campylobacter jejuni respectively*: tests are negative, demonstrating the specificity of the reaction.
**Figure 2** shows photographic images of the cardboards of a second preferred version of the diagnostic kit according to the invention, with the results of the latex agglutination test for the detection of high pathogenicity *H*. *pylori* strains with, in which the following results are visible:
   well 1: agglutination obtained with latex sensitized with anti-CagA;
   wells 2 and 3: agglutination obtained with latex sensitized with antibodies anti-cag antigens;
   well 4: negative control consisting of a cag-negative strain;
**Figure 3** shows the photographic images of the cardboards of the second preferred embodiment of the diagnostic kit according to the invention, with the results of the latex agglutination test for the detection of high pathogenicity *H. pylori* strains, in which the following results are visible:
   well 1: agglutination of a latex sensitized with antibodies anti-Cag antigens with two gastric biopsies from the same patient;
   well 2: a negative case, gastric mucus obtained from a patient a non-infected patient.

### Latex test of gastric biopsies for the diagnosis of infection with Helicobacter pylori expressing CagA

### 1. Latex sensitized with anti-CagA antiserum

### Patients and Methods

41 patients who underwent esophagogastroduodenoscopy for dyspepsia were tested. From each patient four biopsies from the antrum - for the latex agglutination, the rapid urease test, the standard culture and histology, and the modified Giemsa stain (for bacteriological examination) and three biopsies from the body/fundus - for latex agglutination, culture and histology/bacterioscopic examination were taken. A sample of blood from which the serum was separated was also taken from patients, the sample being stored at -20°C until the time of the assay. The sera of patients found to be infected have been then examined for the presence of anti-CagA antibodies with a commercially available ELISA method: "CagA IgG ELISA kit", provided by Genesis Diagnostics, Littleport, UK (96% sensitivity and 97% specificity, inter-assay coefficient of variation <12%).

For the execution of the latex test, the biopsy from the antrum and that from the body were diluted together in 100 µL of a 0.1% solution of dithiothreitol in PBS pH 7.4 (to dissolve the gastric mucus); therefore, approximately 15 µl of dissolved mucus was distributed on three different areas of a cardboard sheet with black background for agglutination. This operation was performed in duplicate, using two sets of six "wells" cardboard sheets.

To the first sample of the first set of cardboard sheets a drop of sensitized latex containing antibodies to surface antigens of *H. pylori* (LHP) was added; to the second sample one drop of LHP and a drop of a suspension of *H. pylori* CCUG 17874 (type strain, CagA-positive) containing 106 organisms per ml in PBS with 1% formalin (positive control) was added, and to the third sample a drop of latex sensitized with rabbit hyperimmune serum (negative control) was added, and mixed thoroughly with a stick. In case of positivity, a clear agglutination was observed in the first two reaction areas, in less than a minute. The negative control remained such even after five minutes.

Then the determination of the presence of the CagA antigen in the second series of cardboard sheets was carried out. The procedure was the same, with the difference that instead of LHP, a suspension of sensitized latex containing anti-CagA antibodies (LCA) was used.

### Results

In 21 out the 41 cases examined, *H. pylori* was isolated in culture and/or observed under the microscope (true positives, TP); in the remaining 20 cases, culture and bacteriological examination were negative (true negatives, TN).

The latex test according to the invention resulted to be positive in 20 of the 21 TP cases (sensitivity = 95.2%) and negative in 18 of the 20 TN cases (specificity = 90.0%), as shown in Table 1 below.

**Table 1. Results of tests with sensitized latex containing antibodies against total antigens of H. pylori (LHP)**

| | | LHP | |
|---|---|---|---|
| | | + | - |
| Culture and/or Bacterioscopy | TP | 20 | 1 |
| | TN | 2 | 18 |
| | | | |

Of the 20 cases in which the latex test was positive for the presence of *H. pylori,* the serological test for anti-CagA antibodies was positive in 12 cases (CTP), and negative in 8 cases (CTN). The latex test for anti-CagA was positive in 10 cases (sensitivity = 83.3%), negative in 7 cases (specificity = 87.5%) as shown in Table 2 below.

**Table 2. Results of tests with sensitized latex containing anti-CagA antibodies (LCA)**

| | | LCA | |
|---|---|---|---|
| | | + | - |
| Serology Anti-CagA | CTP | 10 | 2 |
| | CTN | 1 | 7 |
| | | | |

Unpublished data suggest that the latex agglutination test (LA) could have sensitivity and specificity greater than observed. In fact, some cases in which culture, rapid urease test and histology were negative, the LA test was positive. The search for anti-*H*. *pylori* antibodies in some of these "false-positive" cases had demonstrated the presence of specific serum antibodies.

According to the experience of the Applicant, the 1-2% of patients hosts the coccoid form of *H. pylori* in the stomach, which retains its antigenicity (and therefore can be highlighted by the LA test), but cannot be cultured, and is not detectable by histologic examination.

### 2. Latex sensitized with antiserum against total proteins CagA+ H. pylori adsorbed with a suspension of a CagA⁻ H. pylori strain

Two latex preparations have been compared:
1. Latex sensitized with recombinant anti-CagA antibodies;
2. Latex sensitized with antibodies against total proteins CagA+ *H. pylori* adsorbed with a suspension of a CagA⁻ *H. pylori* strain (G21).

Preliminary tests of the type of those specified in the previous section showed that the latex No. 1 produces an agglutination, with CagA+ strains, not so evident and difficult to be distinguished from that obtained with a CagA⁻ (negative) strain.

A serum obtained as in the case No. 2 contains a much greater concentration IgGs against virulence antigens, compared to serum No. 1, which was obtained by immunizing rabbits with the recombinant CagA protein only. Actually, as already noted, the wild CagA+ strains of possess an insertion, called "pathogenicity island"; that contains about thirty genes involved in virulence, and *cagA* is only one of these. These genes encode peptides with many different functions, which, overall, are used by the bacterium to move the CagA protein in the colonized gastric epithelial cell. These peptides are all immunodominant determinants and stimulate, in the infected individual and in immunized rabbits, a strong production of antibodies. In conclusion, a serum anti-total proteins of a CagA+ strain, adsorbed with a suspension of a CagA⁻ strain, will contain many more antibodies, all directed against antigens involved in virulence.

The comparison between the performances of the two different preparations showed the greater suitability of the preparation No. 2, as is illustrated in the two following tables.

**Table 3 - Latex sensitized with anti-CagA antibodies**

| | | WB | Latex sensitized with anti-CagA antibodies | |
|---|---|---|---|---|
| No. of strains: 20 | CagA expression by Western blot | | + | - |
| | | +15 | 2 | 113 |
| | | -5 | 4 | 1 |

**Table 4 - Latex sensitized with antibodies anti-total proteins of a CagA+ strain adsorbed with a CagA⁻ strain**

| | | WB | Latex sensitized with antibodies anti-total proteins of a CagA+ strain adsorbed with a CagA⁻ strain | |
|---|---|---|---|---|
| No. of strains: 20 | CagA expression by Western blot | | + | - |
| | | +15 | 14 | 1 |
| | | -5 | 1 | 4 |

### 3. Sensitization of a latex suspension with a rabbit serum consisting of antibodies against the antigens encoded by the cag genes

In order to identify those patients infected with *H. pylori* strains with determinants involving the highest risk of serious damage associated with infection by Helicobacter, a latex coated with antibodies against all of the peptides encoded by *cag* genes, and not only by the *cagA* gene, was prepared.

In order to immunize rabbits the strain G33 (CagA+ and VacA+) described in the publication by Xiang et al. was used (Z. Xiang et al., Analysis of Expression of CagA and VacA Virulence Factors in 43 Strains of Helicobacter pylori Reveals that Clinical Isolates Can Be Divided into Two Major Types and that CagA is not Necessary for Expression of the Vacuolating Cytotoxin, Infect Immun Jan. 1995, 63: 94-98), as isolated from a patient with chronic gastritis of high degree, fairly active, with severe atrophy and moderate intestinal metaplasia of fundus. This pathological condition exposes the patient to a higher risk of developing gastric cancer. The characteristics of the strain are reported in the cited publication.

The animal was immunized intravenously (through the marginal ear vein) with seven doses of a suspension of strain G33, each consisting of approximately 10¹⁰ microorganisms per ml in PBS with 0.5% formalin. The immunization was carried out every 3-4 days. After the V dose, small bleedings were performed and the rabbit was finally deprived of all the blood that was possible to obtain (about 50 ml), and then the rabbit was sacrificed when the level of serum agglutinating antibodies had reached the value 1:2000. After sensitizing latex with the serum obtained, and after testing the sensitivity and specificity thereof, the anti-G33 serum was adsorbed with the *H. pylori* G21 strain, non cytotoxic and free of the cag island (Xiang et al., ibidem).

The adsorption was carried out in the following way: G21 was inoculated on ten agar-blood plates, which were incubated in a microaerobic atmosphere at 37°C for 72 hours. The bacterial film was then collected and washed twice with PBS; 1 g of wet paste was then mixed thoroughly with 0.5 ml of anti-G33 serum (final dilution 1:4 in PBS). The mixture was placed to incubate in normal air at 37°C for two hours, with frequent agitation in Vortex; subsequently, it was placed to incubate at 4°C overnight (in order to allow adsorption of incomplete antibodies).

Finally, the mixture was centrifuged at 4000 *g* at 4°C for 20 min and the adsorbed serum was collected and used for sensitizing a latex suspension. The latex so sensitized was then used to identify cag positive strains cultures on plate and in gastric biopsies.

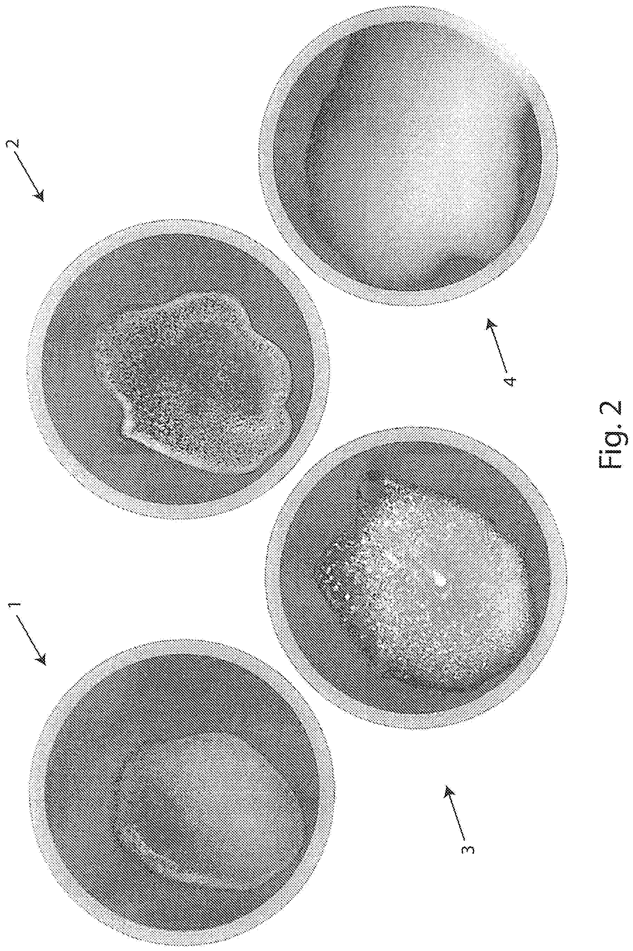

In **Figure 2** of the accompanying drawings agglutination obtained with latex sensitized with anti-CagA (upper left) and with anti-bodies anti-Cag antigens (top right and bottom left; at bottom right negative control consisting of a cag-negative strain) are observed.

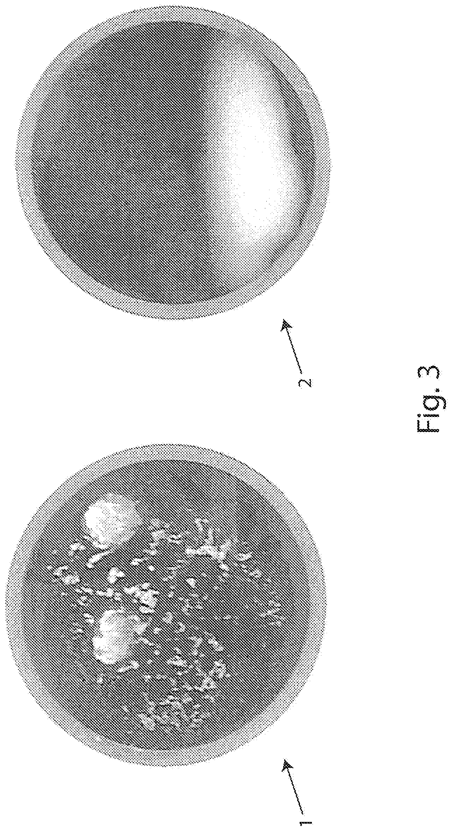

In **Figure 3** a positive result may be seen, to the left (agglutination of latex sensitized with Cag anti-antigens with two gastric biopsies from the same patient) and a negative case, to the right (gastric mucus obtained from a non-infected patient.

The present invention has been disclosed with particular reference to some embodiments thereof, but it should be understood that modifications and changes may be made by the persons skilled in the art without departing from the scope of the invention as defined in the appended claims.

## Claims

1. A method for rapid *in vitro* diagnosis of infection by highly pathogenic strains of Helicobacter pylori comprising the following steps:
a) contacting a suspension of latex particles, sensitized with an antiserum against total proteins of CagA-positive *H. pylori* from which the other anti-bodies against *H. pylori* antigens have been separated, with a biological sample to be analyzed for the presence of the CagA protein antigen, or of protein antigens encoded by the cag genes of *H. pylori,* and mixing said suspension with said biological sample;
b) visually detecting the possible agglutination of the mixture resulting from the previous step;
wherein the agglutination of said mixture shows the presence, in the biological sample under examination, of highly pathogenic *Helicobacter pylori* strains, which express the CagA protein or more proteins encoded by the cag genes.

2. A diagnostic method according to claim 1, wherein said biological sample consists in gastric mucus, stools or in a biopsy specimen of the gastric mucosa.

3. A diagnostic method according to claims 1 o 2, wherein said step a) consists of contacting a suspension of latex particles sensitized with an antiserum against total proteins of CagA-positive *H. pylori* and subsequently adsorbed with a strain of CagA-negative *H. pylori* with a biological sample to be analyzed for the presence of the CagA protein antigen or of protein antigens encoded by *cag* genes, and mixing said suspension with said biological sample.

4. A diagnostic method according to claim 3, wherein said visual detection b) is carried out with the aid of a positive control sample consisting of a suspension of CagA-positive *H. pylori,* which is subjected, as the biological sample to be analyzed, to operations corresponding to steps a) and b).

5. A diagnostic method according to claim 4, wherein said visual detection b) is carried out with the aid of a negative control sample consisting of a suspension of latex particles sensitized or not sensitized with hyperimmune serum, which is used in place of the suspension of step a) when subjecting the biological sample to be analyzed to operations corresponding to the steps a) and b).

6. A diagnostic method according to claims 3 or 4, further comprising the following steps:
c) contacting a suspension of latex particles, sensitized with antiserum against total *H. pylori* antigens, with a biological sample to be analysed for the presence of *H. pylori* antigens, and mixing said suspension with said biological sample; and
d) visually detecting the possible agglutination of the mixture resulting from the previous step;
wherein the agglutination of said mixture shows the presence, in the biological sample under examination, of *Helicobacter pylori* strains, and
the combined performance of the mentioned operations a), b, c) and d) allows detecting both infection by *H. pylori* and the presence of highly pathogenic *Helicobacter pylori* strains.

7. A diagnostic kit for rapid *in vitro* diagnosis of infection by highly pathogenic strains of *Helicobacter pylori* comprising the following components:
A. a suspension of latex particles, sensitized with an antiserum against CagA-positive *H. pylori* from which the other antibodies against *H. pylori* antigens have been separated, in an aqueous solution such as phosphate-buffered saline (PBS) or PBS-formalin, containing an antimicrobial agent, such as merthiolate or sodium azide;
B. an aqueous solution such as PBS or PBS-formalin, containing a mucus-fluidifying agent, such as dithiothreitol, and an antimicrobial agent, such as merthiolate or sodium azide;
C. some cardboard sheets with dark disks, on which the test is to be performed.

8. A diagnostic kit according to claim 7, wherein said latex particles are sensitized with an antiserum against total proteins of CagA-positive *H. pylori* and subsequently adsorbed with a strain of CagA-negative *H. pylori.*

9. A diagnostic kit according to claim 8, which includes, in addition:
D1. a suspension of CagA-positive *H. pylori* antigens, in an aqueous solution such as PBS or PBS-formalin, and an antimicrobial agent, such as merthiolate or sodium azide, as positive control for CagA-positive *H. pylori.*

10. A diagnostic kit according to claims 8 or 9, which includes, in addition:
D2. a suspension of *H. pylori* antigens, in an aqueous solution such as PBS or PBS-formalin, and an antimicrobial agent, such as merthiolate or sodium azide as a positive control for *H. pylori.*

11. A diagnostic kit according to any one of claims 7-10, wherein said component B contains dithiothreitol in a concentration comprised between 0.05 and 1% by weight.

12. A diagnostic kit according to claim 9, wherein said aqueous solution is a phosphate buffered saline (PBS) or a phosphate buffered saline containing 0.5-1.0% by weight of formalin (PBS-formalin), and said antimicrobial agent is merthiolate in a concentration of about 0.05% by weight or sodium azide in a concentration of about 0.1% by weight.

## Patentansprüche

1. Verfahren zur schnellen *in vitro*-Diagnose einer Infektion durch hochgradig pathogene Stämme von Helicobacter pylori, umfassend die folgenden Schritte:
a) Kontaktieren einer Suspension aus Latexpartikeln, die mit einem Antiserum gegen Gesamtproteine von CagA-positivem *H. pylori* sensibilisiert sind, wovon die anderen Antikörper gegen *H. pylori*-Antigene getrennt worden sind, mit einer biologischen Probe, die auf das Vorhandensein des CagA-Proteinantigens oder von Proteinantigenen, die von den Cag-Genen von *H. pylori* kodiert werden, zu analysieren ist, und Mischen der Suspension mit der biologischen Probe;
b) visuelles Erfassen der möglichen Agglutination der Mischung, die sich aus dem vorherigen Schritt ergibt;
wobei die Agglutination jener Mischung das Vorhandensein von hochgradig pathogenen *Helicobacter pylori*-Stämmen in der untersuchten biologischen Probe zeigt, die das CagA-Protein oder mehrere von den *cag*-Genen kodierte Proteine exprimieren.

2. Diagnoseverfahren nach Anspruch 1, wobei die biologische Probe aus Magenschleim, Stuhl oder aus einer Biopsieprobe der Magenschleimhaut besteht.

3. Diagnostisches Verfahren nach Anspruch 1 oder 2, wobei Schritt a) darin besteht, eine Suspension aus Latexpartikeln, die mit einem Antiserum gegen Gesamtproteine von CagA-positivem *H. pylori* sensibilisiert und anschließend mit einem Stamm von CagA-negativem *H. pylori* adsorbiert wurden, mit einer biologischen Probe, die auf das Vorhandensein des CagA-Proteinantigens oder von Proteinantigenen, die durch *cag*-Gene kodiert werden, zu analysieren ist, in Kontakt zu bringen und die Suspension mit dem biologischen Probens zu mischen.

4. Diagnoseverfahren nach Anspruch 3, wobei der visuelle Nachweis b) mit Hilfe einer Positivkontrollprobe durchgeführt wird, die aus einer Suspension aus CagA-positivem *H. pylori* besteht, die als zu analysierende biologische Probe Operationen entsprechend den Schritten a) und b) unterzogen wird.

5. Diagnoseverfahren nach Anspruch 4, wobei der visuelle Nachweis b) unter Zuhilfenahme einer negativen Kontrollprobe durchgeführt wird, die aus einer Suspension aus Latexpartikeln besteht, die mit hyperimmunem Serum sensibilisiert oder nicht sensibilisiert sind, das anstelle der Suspension von Schritt a) verwendet wird, wenn die zu analysierende biologische Probe einer Operation entsprechend den Schritten a) und b) unterzogen wird.

6. Diagnoseverfahren nach den Ansprüchen 3 oder 4, weiter umfassend die folgenden Schritte:
c) Kontaktieren einer Suspension aus Latexpartikeln, die mit Antiserum gegen gesamte *H. pylori*-Antigene sensibilisiert sind, mit einer biologischen Probe, die auf das Vorhandensein von *H. pylori*-Antigene zu analysieren ist, und Mischen der Suspension mit der biologischen Probe; und
d) visuelles Nachweisen der möglichen Agglutination der Mischung, die sich aus dem vorherigen Schritt ergibt;
wobei die Agglutination der Mischung das Vorhandensein von *Helicobacter-pylori-*Stämmen in der untersuchten biologischen Probe und die kombinierte Durchführung der genannten Operationen a), b, c) und d) den Nachweis sowohl einer Infektion durch *H*. *pylori* als auch des Vorhandenseins von hochgradig pathogenen Helicobacter-pylon-Stämmen ermöglicht.

7. Diagnosekit zur schnellen *in vitro*-Diagnose einer Infektion durch hochgradig pathogene Stämme von *Helicobacter pylori,* umfassend die folgenden Komponenten:
A. eine Suspension aus Latexpartikeln, sensibilisiert mit einem Antiserum gegen CagA-positive *H. pylori,* wovon die anderen Antikörper gegen *H. pylori*-Antigene getrennt wurden, in einer wässrigen Lösung wie etwa phosphatgepufferte Kochsalzlösung (PBS) oder PBS-Formalin, enthaltend ein antimikrobielles Mittel, wie etwa Merthiolat oder Natriumazid;
B. eine wässrige Lösung wie etwa PBS oder PBS-Formalin, die ein Schleimverflüssigungsmittel, wie etwa Dithiothreitol, und ein antimikrobielles Mittel, wie etwa Merthiolat oder Natriumazid, enthält;
C. einige Kartonbögen mit dunklen Scheiben, auf denen der Test durchzurühren ist.

8. Diagnosekit nach Anspruch 7, wobei die Latexpartikel mit einem Antiserum gegen Gesamtproteine von CagA-positivem *H. pylon* sensibilisiert und anschließend mit einem Stamm von CagA-negativem *H. pylori* adsorbiert werden.

9. Diagnosekit nach Anspruch 8, der zusätzlich Folgendes beinhaltet:
D1. eine Suspension aus CagA-positiven *H. pylori*-Antigenen in einer wässrigen Lösung wie etwa PBS oder PBS-Formalin und ein antimikrobielles Mittel wie etwa Merthiolat oder Natriumazid als positive Kontrolle für CagA-positive *H. pylori.*

10. Diagnosekit nach den Ansprüchen 8 oder 9, der zusätzlich Folgendes beinhaltet:
D2. eine Suspension aus *H. pylori*-Antigenen in einer wässrigen Lösung wie etwa PBS oder PBS-Formalin und ein antimikrobielles Mittel wie etwa Merthiolat oder Natriumazid als positive Kontrolle für *H. pylori.*

11. Diagnosekit nach einem der Ansprüche 7 bis 10, wobei die Komponente B Dithiothreitol in einer Konzentration zwischen 0,05 und 1 Gew.-% enthält.

12. Diagnosekit nach Anspruch 9, wobei die wässrige Lösung eine phosphatgepufferte Kochsalzlösung (PBS) oder eine phosphatgepufferte Kochsalzlösung ist, die 0,5 bis 1,0 Gew.-% Formalin (PBS-Formalin) enthält, und das antimikrobielle Mittel Merthiolat in einer Konzentration von etwa 0,05 Gew.-% oder Natriumazid in einer Konzentration von etwa 0,1 Gew.-% ist.

## Revendications

1. Procédé pour le diagnostic *in vitro* rapide d'une infection par des souches hautement pathogènes de Helicobacter pylori comprenant les étapes suivantes :
a) la mise en contact d'une suspension de particules de latex, sensibilisées avec un antisérum dirigé contre les protéines totales de *H. pylori* CagA-positif duquel les autres anticorps dirigés contre des antigènes de *H. pylori* ont été séparés, avec un échantillon biologique à analyser pour la présence de l'antigène protéique CagA, ou des antigènes protéiques codés par les gènes *cag* de *H. pylori,* et le mélange de ladite suspension avec ledit échantillon biologique ;
b) la détection visuelle de l'agglutination possible du mélange résultant de l'étape précédente ;
dans lequel l'agglutination dudit mélange montre la présence, dans l'échantillon biologique en cours d'examen, de souches hautement pathogènes de *Helicobacter pylori,* qui expriment la protéine CagA ou plusieurs protéines codées par les gènes *cag.*

2. Procédé de diagnostic selon la revendication 1, dans lequel ledit échantillon biologique consiste en un mucus gastrique, des selles ou en un échantillon de biopsie de la muqueuse gastrique.

3. Procédé de diagnostic selon les revendications 1 ou 2, dans lequel ladite étape a) consiste en la mise en contact d'une suspension de particules de latex sensibilisées avec un antisérum dirigé contre les protéines totales de *H. pylori* CagA-positif et ensuite adsorbées avec une souche de *H. pylori* CagA-négatif avec un échantillon biologique à analyser pour la présence de l'antigène protéique CagA ou des antigènes protéiques codés par les gènes *cag,* et le mélange de ladite suspension avec ledit échantillon biologique.

4. Procédé de diagnostic selon la revendication 3, dans lequel ladite détection visuelle b) est réalisée à l'aide d'un échantillon témoin positif consistant en une suspension de *H. pylori* CagA-positif, qui est soumis, comme l'échantillon biologique à analyser, aux opérations correspondant aux étapes a) et b).

5. Procédé de diagnostic selon la revendication 4, dans lequel ladite détection visuelle b) est réalisée à l'aide d'un échantillon témoin négatif consistant en une suspension de particules de latex sensibilisées ou non sensibilisées avec un hypersérum immun, qui est utilisé à la place de la suspension de l'étape a) lors de la soumission de l'échantillon biologique à analyser aux opérations correspondant aux étapes a) et b).

6. Procédé de diagnostic selon les revendications 3 ou 4, comprenant en outre les étapes suivantes :
c) la mise en contact d'une suspension de particules de latex, sensibilisées avec un antisérum dirigé contre les antigènes totaux de *H. pylori,* avec un échantillon biologique à analyser pour la présence d'antigènes de *H. pylori,* et le mélange de ladite suspension avec ledit échantillon biologique ; et
d) la détection visuelle de l'agglutination possible du mélange résultant de l'étape précédente ;
dans lequel l'agglutination dudit mélange montre la présence, dans l'échantillon biologique en cours d'examen, de souches de *Helicobacter pylori,* et
la performance combinée des opérations a), b, c) et d) mentionnées permet de détecter à la fois une infection par *H. pylori* et la présence de souches hautement pathogènes de *Helicobacter pylori.*

7. Kit de diagnostic pour le diagnostic *in vitro* rapide d'une infection par des souches hautement pathogènes de *Helicobacter pylori* comprenant les composants suivants :
A. une suspension de particules de latex, sensibilisées avec un antisérum dirigé contre *H. pylori* CagA-positif duquel les autres anticorps dirigés contre des antigènes de *H. pylori* ont été séparés, dans une solution aqueuse telle qu'une solution salée tamponnée au phosphate (PBS) ou le PBS-formol, contenant un agent antimicrobien, tel que le merthiolate ou l'azoture de sodium ;
B. un solution aqueuse telle que le PBS ou le PBS-formol, contenant un agent de fluidification de mucus, tel que le dithiothréitol, et un agent antimicrobien, tel que le merthiolate ou l'azoture de sodium ;
C. des feuilles de carton ayant des disques noirs, sur lesquelles le test doit être effectué.

8. Kit de diagnostic selon la revendication 7, dans lequel lesdites particules de latex sont sensibilisées avec un antisérum dirigé contre les protéines totales de *H. pylori* CagA-positif et ensuite adsorbées avec une souche de *H. pylori* CagA-négatif.

9. Kit de diagnostic selon la revendication 8, qui inclut, en plus :
D1. une suspension d'antigènes de *H. pylori* CagA-positif, dans une solution aqueuse telle que le PBS ou le PBS-formol, et d'un agent antimicrobien, tel que le merthiolate ou l'azoture de sodium, en tant que témoin positif pour *H. pylori* CagA-positif.

10. Kit de diagnostic selon les revendications 8 ou 9, qui inclut, en plus :
D2. une suspension d'antigènes de *H. pylori,* dans une solution aqueuse telle que le PBS ou le PBS-formol, et d'un agent antimicrobien, tel que le merthiolate ou l'azoture de sodium en tant que témoin positif pour *H. pylori.*

11. Kit de diagnostic selon l'une quelconque des revendications 7 à 10, dans lequel ledit composant B contient du dithiothréitol à une concentration comprise entre 0,05 à 1 % en poids.

12. Kit de diagnostic selon la revendication 9, dans lequel ladite solution aqueuse est une solution salée tamponnée au phosphate (PBS) ou une solution salée tamponnée au phosphate contenant 0,5 à 1,0% en poids de formol (PBS-formol), et ledit agent antimicrobien est le merthiolate à une concentration d'environ 0,05 % en poids ou l'azoture de sodium à une concentration d'environ 0,1 % en poids.
